# EUROPEAN PATENT APPLICATION

(11) **EP 4 046 638 A1**
(43) Date of publication of application: **24.08.2022**
(21) Application number: 19949392.5
(22) Date of filing: 15.10.2019
(51) Int. Cl.: A61K 31/4709, A61K 31/453, A61K 31/4025, A61K 31/352, A61P 25/04

(54) **USE OF PHENYLQUINOLINONES AND FLAVONOID DERIVATIVES FOR TREATING NEUROPATHIC PAIN**

(71) Applicant: Hangzhou Bio-Sincerity Pharma-tech Co., Ltd., Hangzhou, Zhejiang 311103 (CN)
(72) Inventor: LOU, Jinfang, Hangzhou, Zhejiang 310052 (CN); ZHANG, Fengmin, Hangzhou, Zhejiang 310052 (CN); SHENG, Rong, Hangzhou, Zhejiang 310052 (CN); JIN, Zewu, Hangzhou, Zhejiang 310052 (CN)
(74) Representative: Huang, Liwei
(86) International application number: PCT/CN2019/111269
(87) International publication number: WO 2021/072642

(57) **Abstract**

The present invention provides use of phenylquinolinone deriviative or flavonoid derivative for neuropathic pain. Also, The phenylquinolinone deriviative or flavonoid derivative comprise of their pharmaceutically acceptable salts thereof. The compound of the present invention is a histamine H3 receptor antagonist, has a different mechanism of action from existing drugs, and can be used in the preparation of drugs for treating neuropathic pain diseases. The effect of the compound of the present invention for treating neuropathic pain is superior to that of amitriptyline, gabapentin, pregabalin and carbamazepine. Therefore, the compound of the present invention has a better treatment effect, and has the characteristics of a small dosage, good drug efficacy, high bioavailability and high safety. Compared with opioids, the compound of the present invention has no addiction property, and is a very effective drug for treating neuropathic pain diseases. Therefore, the compound has a great prospect in clinical application. The phenylquinolinone deriviative or flavonoid deriviative has a general structural formula is as following: (I)

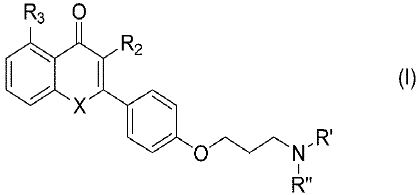

## Description

### The field of the invention

This invention belongs to the technical field of medicine, and relates to the use of phenylquinolinone deriviative or flavonoid derivative in the treatment of neuropathic pain diseases, and more specifically to the use of phenylquinolinone deriviative or flavonoid derivative or their pharmaceutically acceptable salts for preparation of a medicament for the treatment of neuropathic pain diseases. It has the characteristics of small dosage, good therapeutic effect and high safety, and has perfect clinical application prospect.

### The Background of the invention

The World Health Organization (WHO) defines pain as the "fifth most important vital sign" after blood pressure, respiration, pulse and body temperature. Neuropathic pain, also known as nervous pain, neuropathy pain, is a pain caused by peripheral and (or) central nervous system, primary and (or) secondary damage, dysfunction or transitory perturbation. It is a kind of chronic pain, with hyperalgesia, allodynia, paresthesia and spontaneous pain as the main manifestations.

The etiology and clinical manifestations of neuropathic pain performance autoimmune disease (eg, multiple sclerosis), metabolic disease (eg, diabetic neuropathy), infection (eg, postherpetic neuralgia), vascular disease (eg, stroke), nerve compression, nerve trauma and cancer can all lead to neuropathic pain, mainly manifested as spontaneous pain, hyperalgesia, and hyperalgesia. Spontaneous pain is usually described as a constant burning sensation, but can also be classified as intermittent stabbing, tearing, electrocuting, or manifested as dysesthesia or paresthesia. Hyperalgesia is characterized by varying degrees of hypersensitivity to external minor noxious stimuli. Allodynia is a nociceptive response to non-nociceptive stimuli (physiological stimuli).

At present, the main methods of treating neuropathic pain include drug therapy, neuromodulation (spinal cord electrical stimulation, transcutaneous electrical stimulation, acupoint electrical stimulation, etc.), nerve block, nerve pulse radiofrequency, nerve damage (thermocoagulation radiofrequency, chemical damage, Gamma Knife, etc.). For the Vast majority of patients, drug therapy is still the most basic and most popular method. The most Commonly used drugs are tricyclic antidepressants amitriptyline, anticonvulsants gabapentin, pregabalin, carbamazepine, opioids morphine and oxycodone, non-steroidal anti-inflammatory analgesics, NMDA receptor antagonists such as ketamine and methorphan. There are some problems with these drugs mentioned above, which are either inaccurate in drug treatment or have serious adverse reactions. So far, there are no drugs that can effectively reverse the symptoms and function of neuropathic pain. Therefore, it is hoped that reasonable drug treatment can effectively control the symptoms of pain, relieve the pain of patients and improve the quality of life. The research on the drug treatment of neuropathic pain has become an advance subject in the medical field and has important medical and social value.

Invention Patent Application No. 201710488414.7 and PCT/CN2018/089970 provided "Preparation and use of phenylquinolinone and flavonoid derivatives" for the treatment of neurodegenerative diseases, hypoxic-ischemic encephalopathy, Parkinson's syndrome, narcolepsy, epilepsy, ALS disease.

### Summary of the invention

The invention discloses a phenylquinolinone deriviative or flavonoid derivative used in the treatment of neuropathy pain diseases, the phenylquinolinone deriviative or flavonoid derivative including its pharmaceutical acceptable salt. The said use refers to the phenylquinolinone derivative or flavonoid derivative in the preparation of medicines for treatment of neuropathy pain disease, the derivative has a general formula structure as following:

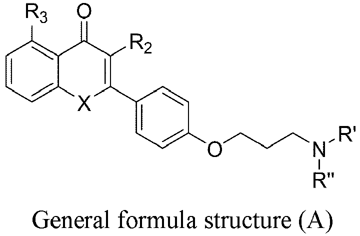

Wherein:
X is 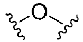 OR 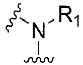 , R₁ is hydrogen or selected from a group consisting of C₁₋₃ alkyl and C₁₋₃ alkoxy;
R₂ is hydrogen or selected from a group consisting of hydroxy and C₁₋₃ alkoxy;
R₃ is hydrogen or selected from a group consisting of hydroxy, C₁₋₃ alkyl, and C₁₋₃ alkoxy;
NR'R" is selected from a group of cyclic amines with 3- to 6-membered carbon atoms and open chain alkylamines with 3- to 6-membered carbon atoms, comprising but not limited to the following segments:

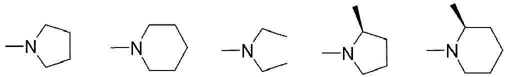

Further, wherein said phenylquinolinone derivative or flavonoid derivative, When X is 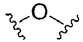 , the derivatives of general formula structure is: A1

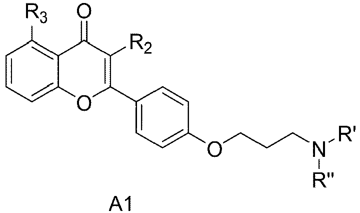

Wherein R₂, R₃, NR'R" are as described above.

When X is 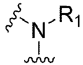 , the derivatives of general formula structure is: A2

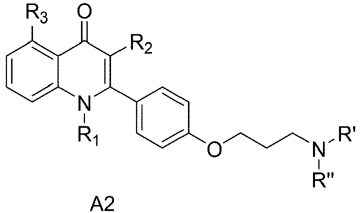

Wherein R₂, R₃, NR'R" are as described above.

Preferred phenylquinolinone deriviative or flavonoid derivative, R₁ is preferably hydrogen, methyl, ethyl and *iso*-propyl; R₂ is preferably hydrogen, hydroxy and methoxy; R₃ is preferably hydrogen, hydroxy and methoxy.

Specifically, preferred compounds (or pharmaceutically acceptable salts of compounds) of phenylquinolinone deriviative and flavonoid derivative are selected from, but not limited to the compounds shown in Table 1.

### Table1 Preferred compounds of phenylquinolinone deriviative and flavonoid derivative

Entry Structure Entry Structure

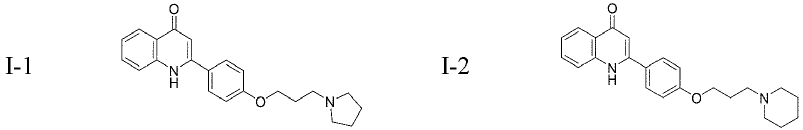

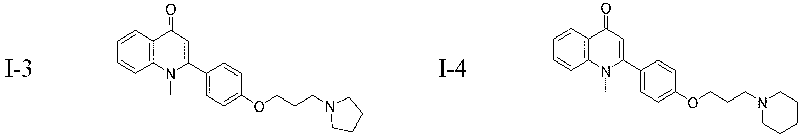

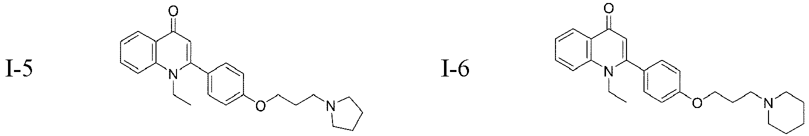

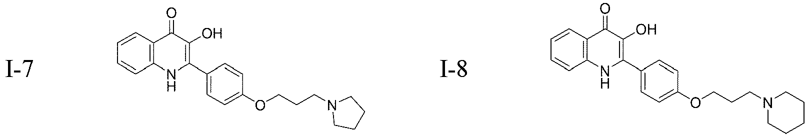

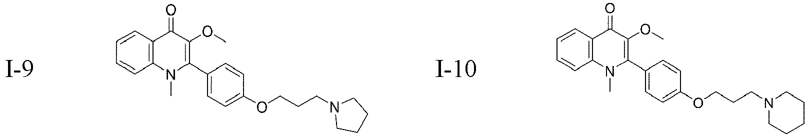

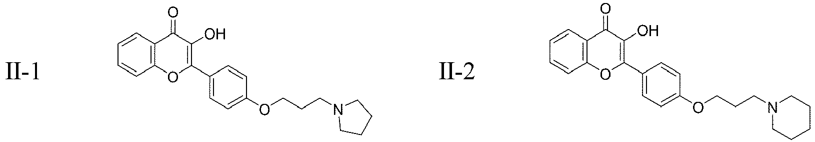

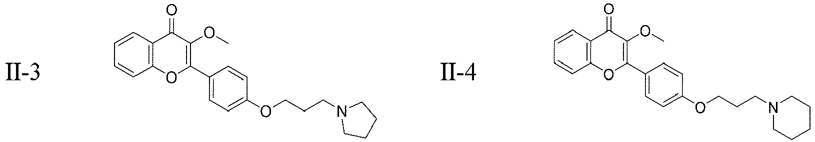

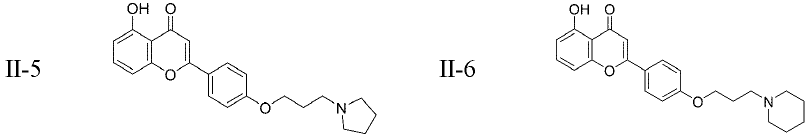

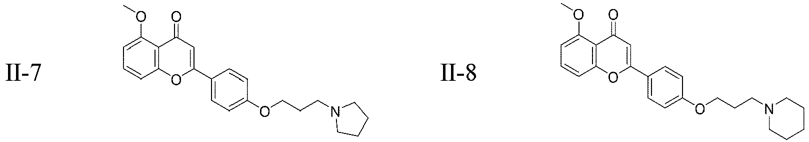

The pharmaceutically acceptable salts of the compounds are hydrochloride, sulfate, phosphate, hydrobromide, hydroiodide, carbonate, citrate, malate, tartrate, oxalate, lactate, malonate, succinate, glutarate, ketoglutarate, ascorbate, fumarate, maleate, methicone sulfonate, one of *p*-toluenesulfonate or benzenesulfonate;

"Alkyl" described herein includes but is not limited to methyl, ethyl, *n*-propyl, isopropyl; "Alkoxyl" includes but is not limited to methoxy, ethoxy, *n*-propoxyl, *iso*-propoxyl. "Alkoxyl" also includes substituted alkoxyl, which may be optionally substituted one or more times with halogen.

The neuropathic pain disease is one of peripheral neuropathic pain or central neuropathic pain.

The peripheral neuropathic pain is trigeminal neuralgia, glossopharyngeal neuralgia, acute or chronic inflammatory demyelinating polyneuralgia, alcoholic polyneuralgia, chemotherapy-induced polyneuralgia, complex area Pain syndrome, entrapment neuralgia (eg, carpal tunnel syndrome), HIV sensory neuralgia, iatrogenic neuralgia (eg, post-mastectomy pain), tumor compression or infiltrating neuralgia, dystrophic-related neuralgia, One of diabetic neuralgia, phantom limb pain, post-herpetic neuralgia, post-radiotherapy plexopathy, radiculopathy (cervical, thoracic or lumbosacral), toxic exposure-related neuralgia, and post-traumatic neuralgia.

The central neuropathic pain is post-stroke pain, multiple sclerosis-related pain, Parkinson's disease-related pain, post-traumatic spinal cord injury pain, syringomyelia, post-ischemic myelopathy, compressive myelopathy, HIV spinal cord disease and post-radiation myelopathy.

The pharmaceutical preparations prepared from the compounds of the present invention or their pharmaceutically acceptable salts and pharmaceutically acceptable carriers, diluents or excipients, the routes of administration are oral administration, rectal administration, nasal administration, topical administration route of administration (including buccal, sublingual or transdermal) or parenteral (including subcutaneous, intramuscular, intravenous or intradermal).

The preparation method of the above-mentioned compounds provided is prepared by the following steps. It is to be understood that the scope of the present invention is not limited in any way by the scope of the following examples and preparations.

1. The quinolinone derivatives of the general formula can be synthesized by the following steps:

### (1) Synthesis method of 1-1, 1-2 in compound I (quinolinone derivatives) series (this method is suitable for X = N):

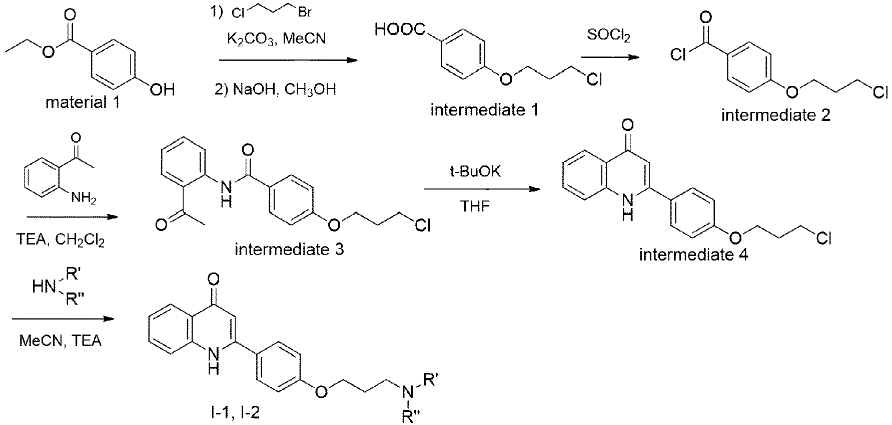

The specific reaction process is:
Firstly, the material 1, 1,3-bromochloropropane and potassium carbonate were dissolved in acetonitrile and heated to reflux, then reacted in NaOH/CH₃OH system to obtain intermediate 1. Secondly, intermediate 1 was refluxed in thionyl chloride solution to obtain intermediate 2. Subsequently, the reaction mixture with o-aminoacetophenone at ambient temperature to obtain intermediate 3, then it reacts with potassium *tert*-butoxide in microwave to obtain intermediate 4. Finally, intermediate 4 refluxed with secondary amine to give the desired products **I-1** and **1-2.**

Note: NR'R" is as described above.

### (2) Synthesis method of 1-3 to 1-6 in compound I (quinolinone derivatives) series (this method is suitable for X=-NR₁-):

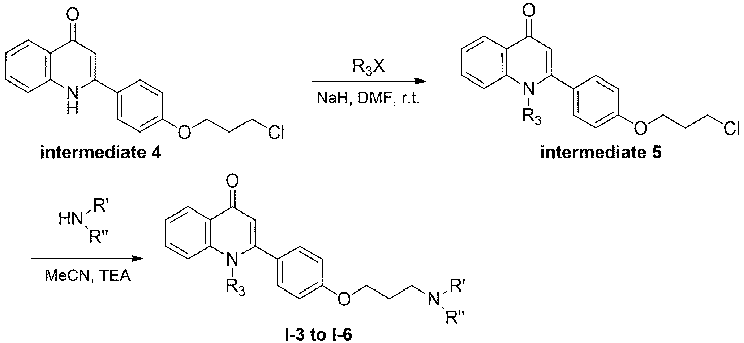

The specific reaction process is:
Intermediate 4 (above-mentioned in step 1) is dissolved in DMF, sodium hydride and halogenated hydrocarbons are added, and the reaction is carried out to obtain intermediate 5. Next, intermediate 5 refluxed with secondary amine and triethylamine to give the desired products **1-3** to **1-6.**

Note: NR'R" is as described above.

### (3) Synthetic method of 1-7 and 1-8 in the series of compound I (quinolinone derivatives) (this method is suitable for X = -NR₁-):

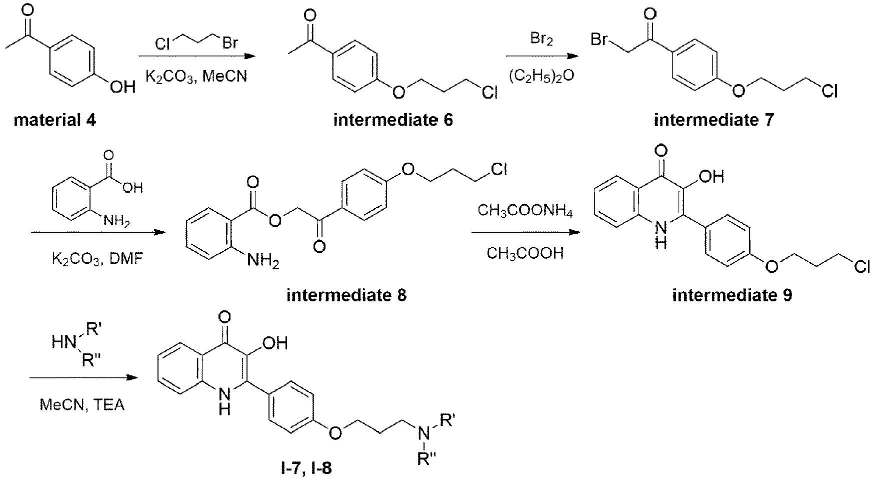

The specific reaction process is:
p-Hydroxyacetophenone (material 4) was dissolved in acetonitrile, 1,3-bromochloropropane and potassium carbonate were added for reflux reaction to obtain intermediate 6, then reacted with bromine under ice bath to obtain bromoketone intermediate 7; Intermediate 7 heated and reacted with anthranilic acid and potassium carbonate in DMF to obtain ester intermediate 8, which is refluxed under ammonium acetate/acetic acid system to obtain intermediate 9. Finally, it is refluxed with secondary amine and triethylamine to give the desired products **1-7, 1-8.**

Note: NR'R" is as described above.

### (4) Synthesis method of 1-9 and 1-10 in compound I (quinolinone derivatives) series (this method is suitable for X = -NR₁-):

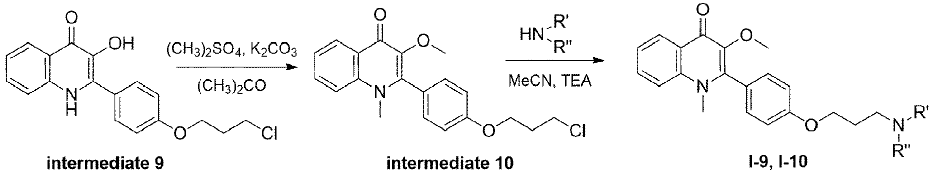

The specific reaction process is:
Intermediate 9 (above-mentioned in steps 3), dimethyl sulfate and potassium carbonate are dissolved in acetone, under reflux reaction obtains intermediate 10. It was refluxed with secondary amine NHR'R" and triethylamine to give the desired products **1-9, 1-10.**

### (5) Synthesis method of II-1 and II-2 in compound II (flavonoid derivatives) series (this method is suitable for X = O):

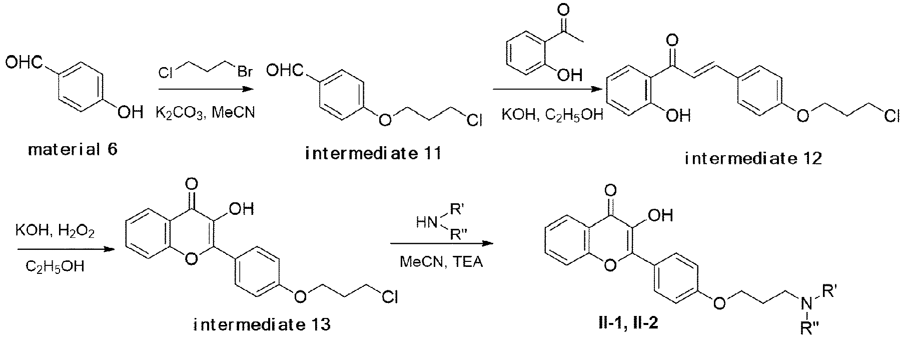

The specific reaction process is:
The material 6 was dissolved in acetonitrile, 1,3-bromochloropropane and potassium carbonate were added, and the intermediate 11 was obtained by refluxing reaction. Intermediate 11 was condensed with p-hydroxyacetophenone to obtain intermediate 12 in potassium hydroxide solution. Then, intermediate 12 passed H₂O₂/KOH system is cyclized to obtain the intermediate 13, which is finally refluxed with secondary amine and triethylamine to give the desired products **II-1, II-2.**

Note: NR'R" is as described above.

### (6) Synthesis method of II-3 and II-4 in compound II (flavonoid derivatives) series (this method is suitable when X = O)

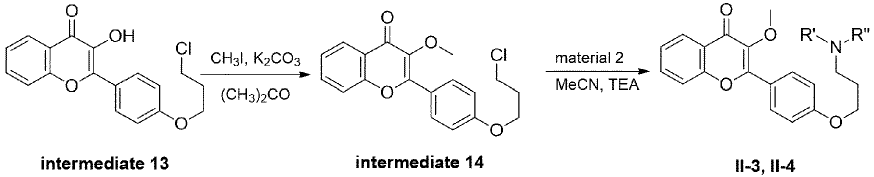

The specific reaction process is:
Intermediate 13 (above-mentioned in step 5), methyl iodide and potassium carbonate are dissolved in acetone. Intermediate 14 is obtained by refluxing reaction, and then the target compound **II-3** and **II-4** are obtained by refluxing reaction with secondary amine and triethylamine.

Note: NR'R" is as described above.

### (7) Synthesis method of II-5 and II-6 in compound II (flavonoid derivatives) series (this method is suitable when X = O)

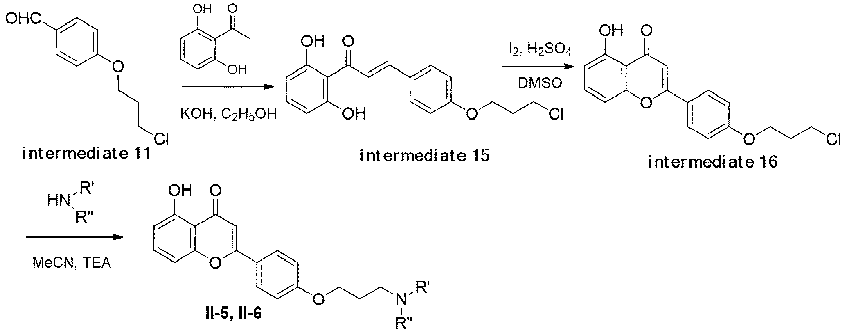

The specific reaction process is:
The intermediate 11 and 2,6-dihydroxyacetophenone were dissolved in ethanol, potassium hydroxide was added, and the reaction was refluxed to obtain the intermediate 15. Then, under the action of iodine and concentrated sulfuric acid, the cyclization reaction was performed to obtain the intermediate 16, which was finally reacted with Secondary amine and triethylamine are refluxed to give the desired products **II-5** and **II-6**.

Note: NR'R" is as described above.

### (8) Synthesis method of II-7 and II-8 in compound II (flavonoid derivatives) series (this method is suitable when X = O)

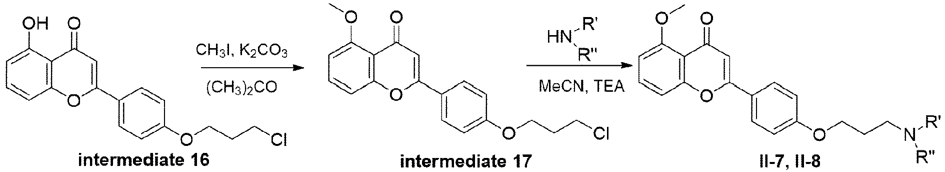

The specific reaction process is:
Intermediate 16(above-mentioned in step 7), potassium iodide and potassium carbonate are dissolved in acetone. The intermediate 17 is obtained by refluxing reaction, compounds **II-7** and **II-8** are obtained by refluxing reaction with secondary amine and triethylamine.

Note: NR'R" is as described above.

Phenylquinolinone deriviative or flavonoid derivative (A) or pharmaceutically acceptable salts, useful for the treatment of neuropathic pain disorders, including peripheral neuropathic pain and central neuropathic pain, peripheral neuropathic pain includes trigeminal neuralgia, glossopharyngeal neuralgia, acute or chronic inflammatory demyelinating polyneuropathy, alcoholic polyneuralgia, chemotherapy-induced polyneuralgia, complex area Pain syndrome, compressive neuralgia (eg, carpal tunnel syndrome), HIV sensory neuralgia, iatrogenic neuralgia (eg, post-mastectomy pain), tumor compression or infiltrating neuralgia, dystrophic-related neuralgia, Diabetic neuralgia, phantom limb pain, postherpetic neuralgia, postradiation plexopathy, radiculopathy (cervical, thoracic, or lumbosacral), toxic exposure-related neuralgia, posttraumatic neuralgia; central neuropathic pain Including post-stroke pain, multiple sclerosis-related pain, Parkinson's disease-related pain, post-traumatic spinal cord injury pain, syringomyelia, post-ischemic myelopathy, compressive myelopathy, HIV myelopathy, and post-radiation myelopathy.

The present invention provides the use of phenylquinolinone deriviative or flavonoid derivative, which is including pharmaceutically acceptable salts, for neuropathic pain. The compound of the present invention is a histamine H3 receptor antagonist, which is different from the action mechanism of the existing drugs, and can be used in the preparation of drugs for treating neuropathic pain diseases. The effect of the compound of the present invention in the treatment of neuropathic pain is better than that of amitriptyline, gabapentin, pregabalin and carbamazepine. Compared with opioids, the compound of the present invention is not addictive, and is a very effective drug for treating neuropathic pain diseases. Therefore, the clinical application prospect is huge.

### Description of figures

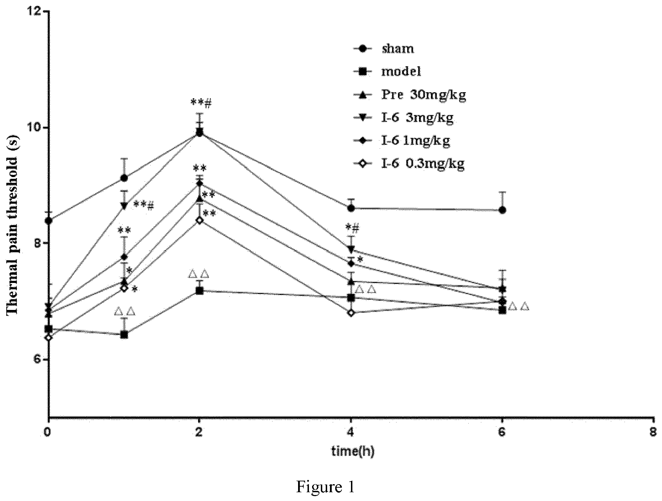

Figure 1 is a single dose of I-6 compound (0.3, 1, 3 mg/kg, PO) and pregabalin (30 mg/kg, PO) on thermal thresholds in PSL rats (Mean±SEM, N=4) . One-way analysis of variance was used, model and sham group were compared, ^{△}, P<0.05, ^{△△}, P<0.01; each administration group was compared with model group, ^{∗}, P<0.05, ^{∗∗}, P<0.01; 1-6 Compared with the Pre group, #, P<0.05, ##, P<0.01.

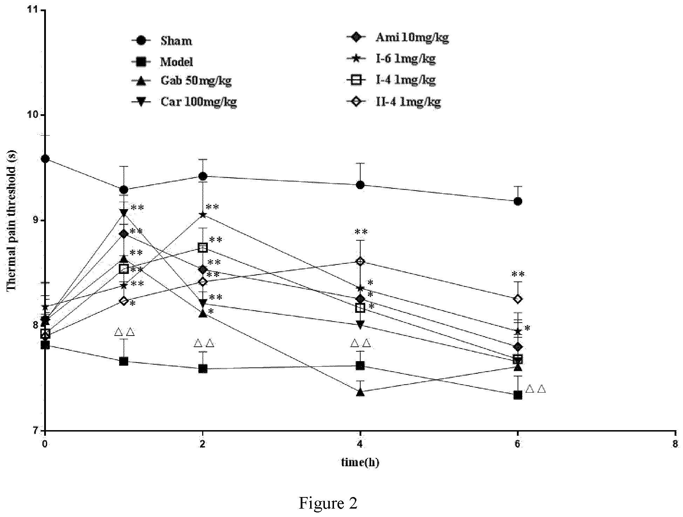

Figure 2 is a single dose of 1-4 (1 mg/kg, PO), 1-6 (1 mg/kg, PO), II-4 (1 mg/kg, PO) and gabapentin (50 mg/kg, PO), Carbamazepine (100 mg/kg, PO), amitriptyline (10 mg/kg, PO) on thermal pain threshold in PSL rats (Mean±SEM, N=4). One-way analysis of variance was used, the comparison between model and sham group, ^{△} , P<0.05, ^{△ △} , P<0.01; the comparison between each administration group and model group, ^{∗}, P<0.05, ^{∗∗}, P<0.01.

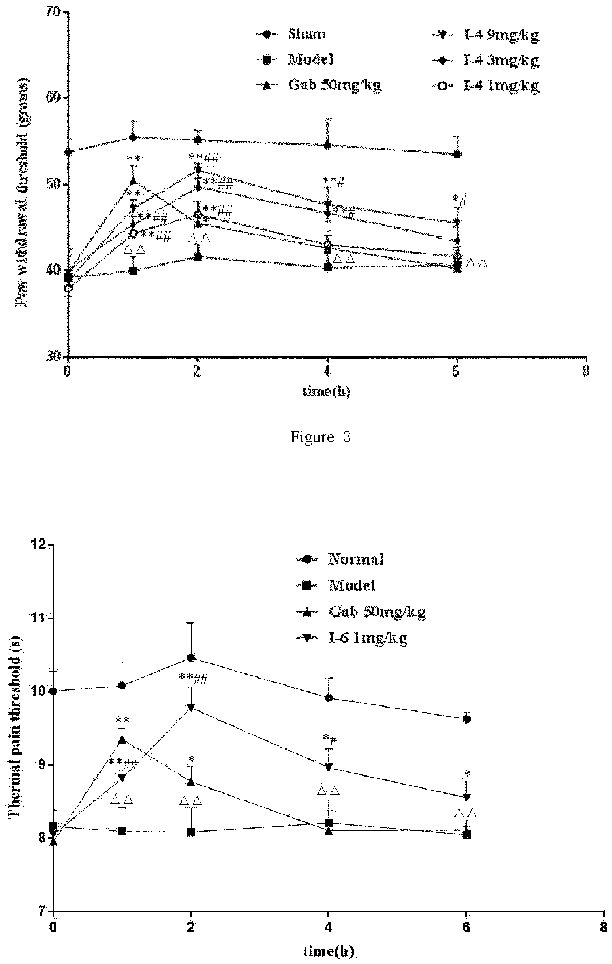

Figure 3 is a single dose of 1-4 compound (1, 3, 9 mg/kg, PO) and gabapentin (Gab, 50 mg/kg, PO) on the mechanical pain threshold of CCI rats (Mean±SEM, N=4 ). Using one-way analysis of variance, the comparison between model and sham group, ^{△}, P<0.05, ^{△△}, P<0.01; the comparison between each administration group and model group, ^{∗}, P<0.05, ^{∗∗}, P<0.01, 1-4 Compared with Gab group, #, P<0.05, ##, P<0.01.

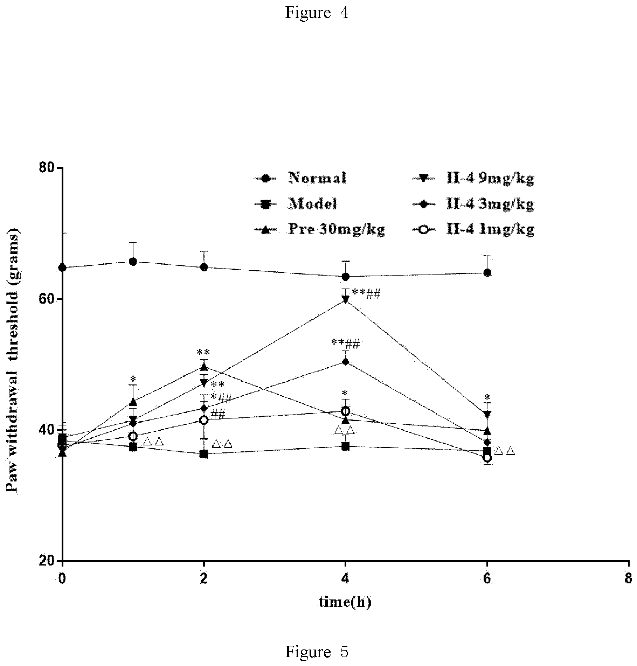

Figure 4 shows the thermal pain threshold (Mean±SEM, N=4) of STZ-induced diabetic neuropathic pain rats after single administration of 1-6 compound (1 mg/kg, P.O.) and gabapentin (50 mg/kg, P.O.). One-way analysis of variance was used, model and Normal group were compared, ^{△}, P<0.05, ^{△△}, P<0.01; each administration group was compared with model group, ^{∗}, P<0.05, ^{∗∗}, P<0.01, 1-6 Compound and gabapentin group, #, P<0.05, ##, P<0.01.
Figure 5 is a single dose of compound II-4 (1, 3, 9 mg/kg, PO) and Pre (30 mg/kg, PO) on the mechanical pain threshold of OXA-induced neuropathic pain in rats (Mean ± SEM, N =4). One-way analysis of variance, model and Normal group comparison, ^{△}, P<0.05, ^{△△}, P<0.01; each administration group compared with model group, ^{∗}, P<0.05, ^{∗∗}, P<0.01, II-4 Compared with the Pre group, #, P<0.05, ##, P<0.01.

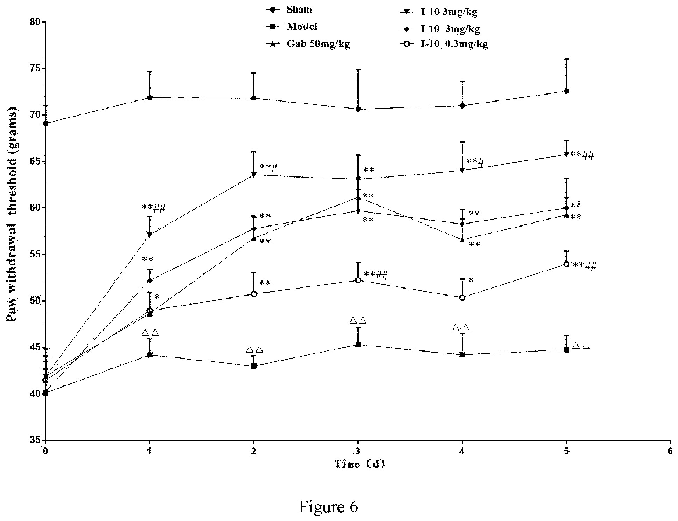

Figure 6 is a continuous administration of 5d, 1-10 compound (0.3, 1, 3 mg/kg, PO) and gabapentin (Gab, 50 mg/kg, PO) on the mechanical pain threshold of CCI rats (Mean ± SEM, N = 4). One-way analysis of variance was used, model and sham group were compared, ^{△}, P<0.05, ^{△△}, P<0.01; each administration group was compared with model group, ^{∗}, P<0.05, ^{∗∗}, P<0.01, 1-10 Compared with Gab group, #, P<0.05, ##, P<0.01.

### Specific implementation way

Further description will be given below in conjunction with the accompanying drawings and embodiments. The specific examples below are included for purposes of illustration and should not be construed as limiting the scope of the invention. It is further understood that, after reading the material taught about the invention, a person skilled in the field may make any modification or modification of the invention, and that such equivalent forms fall within the limits of the claims attached to this application.

### Example 1. Synthesis of I-1, I-2 in Class I Compounds (quinolinones)

### Step 1: 4-(3-Chloropropoxy)benzoic acid (Intermediate 1)

Ethyl p-hydroxybenzoate II-1 (12 g, 72 mmol), 1,3-bromochloropropane (14.2 mL, 144 mmol) and K₂CO₃ (20 g, 144 mmol) were dissolved in 100 mL of acetonitrile and heated to reflux for 12 h. After the reaction mixture filtered to remove excess K₂CO₃, concentrated in vacuo, we obtain colorless oil. The colorless oil was added 15 mL of 6 N NaOH solution and 30 mL of CH₃OH, then refluxing reaction for 1 h. When the reaction mixture turned to clarified solution, cooled to ambient temperature, acidified to pH = 2 with 2 N hydrochloric acid. At the same time, a large amount of white solid was precipitated, then the reaction mixture undergo filtered, washed with water, dried to obtain 14.5 g of white solid powder, Yield = 94%; ¹H NMR (500 MHz, CDCl₃): δ 8.08 (d, *J=* 8.5 Hz, 2H), 6.96 (d, *J=* 8.5 Hz, 2H), 4.21 (t, *J* = 6.0 Hz, 2H), 3.77 (t, *J* = 6.5 Hz, 2H), 2.30-2.25 (m, 2H); ESI-MS: *m*/*z* =215 [M⁺H]⁺_{∘}

### Step 2: N-(2-Acetylphenyl)-4-(3-chloropropoxy)benzamide (Intermediate 3)

Intermediate 1 (5.0 g, 23 mmol) was refluxed in 10 mL SOCl₂ for 1 h, and 1~2 drops of DMF were added to the reaction mixture. After the reaction mixture had been concentrated in vacuo to remove excess SOCl₂, we obtain colorless oil (Intermediate 2). *o*-aminophenone (2.83 g, 21 mmol) was dissolved in 15 mL anhydrous CH₂Cl₂ and 6.5 mL anhydrous TEA, and intermediate 2 was added slowly at 0°C. After dropping, the reaction was continued for 2h at ambient temperature. After the reaction mixture had been filtered, concentrated in vacuo. The residue was purified by Silica gel chromatography (Petroleum ether: EtOAc = 10: 1) to give Intermediate 3 as white solid 5.0g, Yield = 72%; ¹H NMR (500 MHz, CDCl₃): δ 12.65 (s, 1H), 8.11 (d, *J=* 9.0 Hz, 1H), 8.05 (d, *J=* 9.0 Hz, 2H), 7.97 (dd, *J=* 8.0, 1.5 Hz, 1H), 7.64 (t, *J=* 8.0 Hz, 1H), 7.16 (t, *J=* 8.0 Hz, 1H), 7.02 (d, *J=* 9.0 Hz, 2H), 4.21 (t, *J=* 6.0 Hz, 2H), 3.78 (t, *J=* 6.5 Hz, 2H), 2.73 (m, 3H), 2.31-2.26 (m, 2H); ESI-MS: *m*/*z* =332 [M+H]⁺_{∘}

### Step 3: 2-(4-(3-Chloropropoxy)phenyl)quinolin-4(1H)-one (Intermediate 4)

Intermediate 3 (995 mg, 3.0 mmol), potassium *tert*-butoxide (1.68 g, 15 mmol) were dissolved in 15 mL of THF. After The reaction mixture was microwaved at 110 °C for 20 min in a sealed container. When the reaction mixture cooled to ambient temperature, pooed into 100 mL ice water, acidified to pH = 5~6 with 2 N hydrochloric acid. At the same time, a large amount of yellow solid was precipitated. Then the reaction mixture undergo filtered, washed with water and a mixture of acetone with CH₂Cl₂ (1:1) obtained Intermediate 4 (750mg), Yield = 80%; ¹H NMR (500 MHz, CDCl₃): δ 11.63 (s, 1H), 8.10 (dd, *J=* 8.0, 1.0 Hz, 1H), 7.83 (d, *J* = 9.0 Hz, 2H), 7.78 (d, *J=* 8.0, 1H), 7.68 (t, *J=* 7.5 Hz, 1H), 7.35 (t, *J=* 7.5 Hz, 1H), 7.17 (d, *J* = 9.0 Hz, 2H), 6.33 (s, 1H), 4.21 (t, *J=* 6.0 Hz, 2H), 3.84 (t, *J=* 6.5 Hz, 2H), 2.24-2.18 (m, 2H); ESI-MS: *m*/*z* =314 [M+H]⁺_{∘}

### Step 4: 2-(4-(3-(pyrrolidine-1-yl)propoxy)phenyl)quinoline-4(1H)-one (1-1)

Intermediate 4 (60 mg, 0.19 mmol) was dissolved in 3 mL of acetonitrile, 41 mg (0.57 mmol) of pyrrolidine and 96 mg (0.96 mmol) of TEA were added dropwise, and heated to reflux for overnight. After the reaction mixture had been cooled and concentrated in vacuo. The residue was purified by Silica gel chromatography (Petroleum ether: EtOAc: TEA = 1: 5: 0.1) provided I-1 as yellow solid 40 mg, Yield = 60%; ¹H NMR (500 MHz, CDCl₃): δ 8.33 (d, *J=* 9.0 Hz, 1H), 7.86 (d, *J=* 8.0 Hz, 1H), 7.62 (t, *J=* 7.5 Hz, 1H), 7.55 (t, *J=* 8.5 Hz, 2H), 7.34 (t, *J=* 7.5 Hz, 1H), 6.76 (d, *J=* 8.5 Hz, 2H), 6.38 (s, 1H), 3.91 (t, *J=* 6.0 Hz, 2H), 2.95-2.88 (m, 6H), 2.12-2.07 (m, 2H), 1.98-1.93 (m, 2H); ¹³C NMR (125 MHz, CDCl₃): δ 174.19, 155.44, 146.00, 136.03, 127.08, 124.08, 122.23, 120.66, 120.41, 118.90, 113.92, 109.83, 102.77, 60.93, 48.61, 47.68, 22.01, 18.61; ESI-MS: *m*/*z* =349 [M+H]⁺_{∘}

### 2-(4-(3-(piperidin-1-yl)propoxy)phenyl)quinolin-4(1H)-one (1-2)

The preparation method was the same as that of compound 1-1, except that piperidine is used instead of pyrrolidine to obtain yellow solid. Yield = 75%; ¹H NMR (500 MHz, CDCl₃): δ 8.33 (d, *J=* 8.5 Hz, 1H), 7.79 (d, *J=* 8.5 Hz, 1H), 7.62 (t, *J=* 7.5 Hz, 1H), 7.59 (t, *J=* 8.5 Hz, 2H), 7.34 (t, *J=* 7.5 Hz, 1H), 6.84 (d, *J=* 8.5 Hz, 2H), 6.39 (s, 1H), 3.95 (t, *J=* 6.0 Hz, 2H), 2.59-2.54 (m, 6H), 2.04-1.99 (m, 2H), 1.70-1.66 (m, 4H), 1.51-1.48 (m, 2H); ¹³C NMR (125 MHz, CDCl₃): δ 174.16, 155.86, 145.64, 135.72, 127.17, 123.84, 121.89, 120.82, 120.43, 118.94, 113.68, 110.07, 102.82, 61.52, 50.78, 49.56, 21.23, 20.50, 19.15; ESI-MS: *m*/*z* =363 [M⁺H]⁺_{∘}

### Example 2. Synthesis of I-3~I-6 in class I compounds (quinolinones)

### Step 1: 2-(4-(3-Chloropropoxy)phenyl)-1-methylquinolin-4(1H)-one (Intermediate 5a)

Intermediate 4 (276 mg, 0.88 mmol) was dissolved in 5 mL of DMF, 42 mg of NaH (60%, 1.05 mmol) was added, stirred at room temperature for 30 min, then iodomethane (138 mg, 0.97 mmol) was added, the reaction mixture heated to 35 °C for 30 min. The reaction solution was poured into 50 mL of H₂O, extracted with EtOAc, washed with water, washed with saturated NaCl, and dried over anhydrous Na₂SO₄. After recovering the solvent, it was separated by column chromatography (petroleum ether: EtOAc = 10: 1) to obtain 250 mg of white solid. Yield = 87%; ¹H NMR (500 MHz, CDCl₃): δ 8.17 (d, *J=* 8.0 Hz, 1H), 8.10-8.08 (m, 3H), 7.71 (d, *J=* 8.0, 1H), 7.48 (t, *J=* 7.5 Hz, 1H), 7.14 (s, 1H), 7.05 (d, *J=* 9.0 Hz, 2H), 4.21 (t, *J=* 6.0 Hz, 2H), 4.12 (s, 3H), 3.79 (t, *J=* 6.5 Hz, 2H), 2.31-2.26 (m, 2H); ESI-MS: *m*/*z* =328 [M+H]⁺_{∘}

### 2-(4-(3-Chloropropoxy)phenyl)-1-ethylquinolin-4(1H)-one (Intermediate 5b)

The preparation method was the same as that of compound intermediate 5a, and bromoethane is used instead of iodomethane to obtain yellow solid. Yield = 67%; ¹H NMR (500 MHz, CDCl₃): δ 8.21 (d, *J=* 8.0 Hz, 1H), 8.09-8.07 (m, 3H), 7.71 (d, *J=* 8.0, 1H), 7.48 (t, *J=* 7.5 Hz, 1H), 7.12 (s, 1H), 7.05 (d, *J=* 9.0 Hz, 2H), 4.37 (q, *J=* 7.0 Hz, 2H), 4.21 (t, *J=* 6.0 Hz, 2H), 3.79 (t, *J=* 6.5 Hz, 2H), 2.31-2.26 (m, 2H), 1.62 (s, 3H); ESI-MS: *m*/*z* =342 [M+H]⁺_{∘}

### Step 2: 1-methyl-2-(4-(3-(pyrrolidin-1-yl)propoxy)phenyl)quinolin-4(1H)-one (1-3)

The preparation method was the same as that of compound 1-1, except that intermediate 5a is used instead of intermediate 4, and the secondary amine is pyrrolidine to obtain white solid 1-3, yield = 74%; ¹H NMR (500 MHz, CDCl₃): δ 8.17 (d, *J=* 8.0 Hz, 1H), 8.09-8.06 (m, 3H), 7.70 (t, *J=* 8.0, 1H), 7.47 (t, *J=* 7.5 Hz, 1H), 7.14 (s, 1H), 7.04 (d, *J=* 9.0 Hz, 2H), 4.13 (t, *J=* 6.0 Hz, 2H), 4.12 (s, 3H), 2.71 (t, *J=* 6.5 Hz, 2H), 2.61-2.57 (m, 4H), 2.10-2.05 (m, 2H), 1.85-1.80 (m, 4H); ¹³C NMR (100 MHz, CDCl₃): δ 162.70, 160.18, 158.36, 149.15, 132.72, 129.87, 128.95, 128.79, 125.01, 121.57, 120.15, 114.66, 97.40, 66.51, 55.59, 54.28, 53.17, 28.82, 23.44; ESI-MS: *m*/*z* =363 [M+H]⁺_{∘}

### 1-methyl-2-(4-(3-(piperidin-1-yl)propoxy)phenyl)quinolin-4(1H)-one (1-4)

The preparation method was the same as compound 1-1, using intermediate 5a instead of intermediate 4, and using piperidine instead of pyrrolidine for secondary amine to obtain white solid 1-4 with a yield of 71%; ¹H NMR (500 MHz, CDCl₃): δ 8.17 (d, *J* = 8.0 Hz, 1H), 8.09-8.06 (m, 3H), 7.70 (d, *J=* 8.0, 1H), 7.47 (t, *J=* 7.5 Hz, 1H), 7.14 (s, 1H), 7.04 (d, *J=* 9.0 Hz, 2H), 4.12 (s, 3H), 4.11 (t, *J* = 6.0 Hz, 2H), 2.58-2.52 (m, 2H), 2.50-2.44 (m, 4H), 2.08-2.03 (m, 2H), 1.66-1.62 (m, 4H), 1.47-1.45 (m, 2H); ¹³C NMR (100 MHz, CDCl₃): δ 162.70, 160.22, 158.41, 149.18, 132.75, 129.92, 128.99, 128.83, 125.06, 121.61, 120.18, 114.69, 97.46, 66.65, 56.04, 55.64, 54.69, 26.80, 25.97, 24.43; ESI-MS: *m*/*z* =377 [M+H]⁺_{∘}

### 1-ethyl-2-(4-(3-(pyrrolidin-1-yl)propoxy)phenyl)quinolin-4(1H)-one (1-5)

The preparation method was the same as that of compound 1-1, except that intermediate 5b is used instead of intermediate 4, and the secondary amine is pyrrolidine to obtain light yellow solid 1-5. Yield = 74%; ¹H NMR (500 MHz, CDCl₃): δ 8.21 (d, *J* = 8.0 Hz, 1H), 8.07-8.05 (m, 3H), 7.70 (t, *J=* 8.0, 1H), 7.47 (t, *J=* 7.5 Hz, 1H), 7.12 (s, 1H), 7.04 (d, *J=* 9.0 Hz, 2H), 4.37 (q, *J=* 7.0 Hz, 2H), 4.13 (t, *J=* 6.0 Hz, 2H), 2.77-2.75 (m, 2H), 2.71-2.64 (m, 4H), 2.14-2.10 (m, 2H), 1.89-1.85 (m, 4H), 1.62 (t, *J=* 7.0 Hz, 3H); ¹³C NMR (100 MHz, CDCl₃): δ161.98, 160.17, 158.35, 149.25, 132.85, 129.77, 128.96, 128.78, 124.86, 121.69, 120.26, 114.67, 97.95, 66.53, 63.97, 54.27, 53.16, 28.82, 23.46, 14.54; ESI-MS: *m*/*z* =377 [M⁺H]⁺_{∘}

### 1-ethyl-2-(4-(3-(piperidin-1-yl)propoxy)phenyl)quinolin-4(1H)-one (1-6)

The preparation method was the same as that of compound 1-1, except that intermediate 5b is used instead of intermediate 4, and the secondary amine is piperidine to obtain light yellow solid 1-6. Yield = 85%; ¹H NMR (500 MHz, CDCl₃): δ 8.21 (d, *J* = 8.0 Hz, 1H), 8.07-8.05 (m, 3H), 7.70 (t, *J=* 8.0, 1H), 7.47 (t, *J=* 7.5 Hz, 1H), 7.12 (s, 1H), 7.04 (d, *J=* 9.0 Hz, 2H), 4.37 (q, *J=* 7.0 Hz, 2H), 4.11 (t, *J=* 6.0 Hz, 2H), 2.60-2.52 (m, 2H), 2.51-2.44 (m, 4H), 2.10-2.05 (m, 2H), 1.68-1.63 (m, 4H), 1.62 (t, *J=* 7.0 Hz, 3H), 1.50-1.45 (m, 2H); ¹³C NMR (100 MHz, CDCl₃): δ 162.00, 160.18, 158.40, 149.25, 132.85, 129.83, 128.96, 128.81, 124.91, 121.73, 120.26, 114.68, 98.00, 66.63, 64.01, 56.00, 54.66, 26.77, 25.93, 24.41, 14.59; ESI-MS: *m*/*z* =391 [M+H]⁺_{∘}

### Example 3. Synthesis of 1-7 and 1-8 in class I compounds (quinolinones)

### Step 1: 4-(3-Chloropropoxy)acetophenone (Intermediate 6)

p-Hydroxyacetophenone (5.0 g, 36.7 mmol), 1,3-bromochloropropane (7.3 mL, 73.5 mmol) and K₂CO₃ (10 g, 73.5 mmol) were dissolved in 30 mL of acetonitrile and heated to reflux for 10 h. After the reaction mixture filtered to remove excess K₂CO₃, concentrated in vacuo. The residue was purified by column chromatography (petroleum ether: EtOAc = 10:1) to afford 7.6 g of colorless oil. Yield = 98%; ¹H NMR (500 MHz, CDCl₃): δ 7.95 (d, *J=* 9.0 Hz, 2H), 6.95 (d, *J* = 9.0 Hz, 2H), 4.20 (t, *J=* 6.0 Hz, 2H), 3.77 (t, *J=* 6.0 Hz, 2H), 2.56 (s, 3H), 2.29-2.24 (m, 2H); ESI-MS: *m*/*z* =213 [M+H]⁺_{∘}

### Step 2: 2-Bromo-1-(4-(3-chloropropoxy)phenyl)ethanone (Intermediate 7)

Intermediate 6 (2.12 g, 10 mmol) was dissolved in 20 mL of diethyl ether, and Br₂ (0.51 mL, 10 mmol) was slowly added dropwise at 0 °C. After the dropwise addition, the mixture was stirred at ambient temperature for 16 h. After, the reaction solution was poured into saturated NaHCO₃ solution, extracted with ether, the organic layer was dried over anhydrous Na₂SO₄, and separated by column chromatography (petroleum ether: CH₂Cl₂=3:1) to obtain 2.45 g light yellow solid. Yield = 84%; ¹H NMR (500 MHz, CDCl₃): δ 7.97 (d, *J=* 9.0 Hz, 2H), 6.97 (d, *J* = 9.0 Hz, 2H), 4.40 (s, 2H), 4.22 (t, *J* = 6.0 Hz, 2H), 3.77 (t, *J* = 60 Hz, 2H), 2.30-2.25 (m, 2H); ESI-MS: *m*/*z* =291 [M+H]⁺_{∘}

### Step 3: 2-(4-(3-Chloropropoxy)phenyl)-2-oxoethyl-2-aminobenzoate (Intermediate 8)

Anthranilic acid (720 mg, 5.25 mmol), K₂CO₃ (760 mg, 5.5 mmol) were dissolved in 10 mL of DMF, stirred at ambient temperature for 30 min, then intermediate 7 (1.46 g, 5.0 mmol) was added, heated to 50 °C for 3 h. The reaction mixture was poured into 100 mL of water, extracted with EtOAc. The combined organic layer was washed with 1 N NaOH solution and saturated aqueous sodium chloride solution, dried, concentrated in vacuo. The residue was purified by column chromatography (petroleum ether: EtOAc = 3:1) to afford 1.6 g of white solid. Yield = 92%; ¹H NMR (500 MHz, CDCl₃): δ 8.02 (dd, *J=* 8.0, 1.0 Hz, 1H), 7.96 (d, *J=* 8.5 Hz, 2H), 7.32 (t, *J=* 7.5 Hz, 1H), 6.98 (d, *J=* 8.5 Hz, 2H), 6.72-6.68 (m, 2H), 5.49 (s, 2H), 4.22 (t, *J* = 6.0 Hz, 2H), 3.77 (t, *J* = 6.0 Hz, 2H), 2.30-2.25 (m, 2H); ESI-MS: *m*/*z* =348 [M⁺H]⁺_{∘}

### Step 4: 2-(4-(3-Chloropropoxy)phenyl)-3-hydroxyquinolin-4(1H)-one (Intermediate 9)

Intermediate 8 (1.6 g, 4.6 mmol) and ammonium acetate (5.3 g, 69 mmol) were dissolved in 30 mL of acetic acid and heated to reflux for 3 h. The reaction mixture was poured into 250 mL of water, a large amount of solid was precipitated, filtered, washed with water until neutral, and dried to obtain 1.02 g light yellow solid. Yield = 67%; ¹H NMR (500 MHz, CDCl₃): δ 8.32 (d, *J* = 8.5 Hz, 1H), 7.80-7.76 (m, 2H), 7.61-7.57 (m, 2H), 7.32 (t, *J* = 7.5 Hz, 1H), 6.97-6.95 (m, 2H), 6.72-6.68 (m, 2H), 4.12 (t, *J* = 6.0 Hz, 2H), 3.76 (t, *J* = 6.0 Hz, 2H), 2.26-2.23 (m, 2H); ESI-MS: *m*/*z* =330 [M+H]⁺_{∘}

### Step 5: 3-hydroxy-2-(4-(3-(pyrrolidin-1-yl)propoxy)phenyl)quinolin-4(1H)-one (1-7)

The preparation method was the same as that of compound 1-1, and intermediate 9 is used instead of intermediate 4 to obtain beige solid. Yield = 59%; ¹H NMR (500 MHz, DMSO-*d*₆): δ 11.48 (s, 1H), 8.29 (s, 1H), 8.13 (dd, *J* = 8.0, 1.0 Hz, 1H), 7.78 (d, *J* = 9.0 Hz, 2H), 7.73 (d, *J* = 8.5 Hz, 1H), 7.60 (t, *J=* 7.5 Hz, 1H), 7.28 (t, *J=* 7.5 Hz, 1H), 7.13 (d, *J=* 9.0 Hz, 2H), 4.12 (t, *J* = 6.5 Hz, 2H), 2.56 (t, *J* = 7.0 Hz, 2H), 2.46-2.42 (m, 4H), 1.95-1.89 (m, 2H), 1.70-1.67 (m, 4H); ¹³C NMR (125 MHz, DMSO-*d*₆): δ 170.22, 159.80, 138.42, 138.01, 131.81, 131.11, 130.85, 124.85, 124.80, 122.24, 122.15, 118.84, 114.66, 66.54, 54.13, 52.68, 28.63, 23.58; ESI-MS: *m*/*z* =365 [M+H]⁺_{∘}

### 3-hydroxy-2-(4-(3-(piperidin-1-yl)propoxy)phenyl)quinolin-4(1H)-one (1-8)

The preparation method was the same as compound 1-1, using intermediate 9 instead of intermediate 4 and piperidine instead of pyrrolidine to obtain yellow solid. Yield = 67%; ¹H NMR (500 MHz, DMSO-*d*₆): δ 11.47 (s, 1H), 8.28 (s, 1H), 8.12 (dd, *J* = 8.0, 1.0 Hz, 1H), 7.77 (d, *J=* 90 Hz, 2H), 7.72 (d, *J* = 8.5 Hz, 1H), 7.59 (t, *J* = 7.5 Hz, 1H), 7.27 (t, *J* = 7.5 Hz, 1H), 7.12 (d, *J=* 9.0 Hz, 2H), 4.12 (t, *J* = 6.5 Hz, 2H), 2.56 (t, *J* = 7.0 Hz, 2H), 2.46-2.42 (m, 4H), 1.95-1.89 (m, 2H), 1.70-1.67 (m, 4H), 1.51-1.48 (m, 2H); ¹³C NMR (125 MHz, DMSO-*d*₆): δ 170.25, 159.49, 138.43, 138.03, 131.75, 131.17, 130.84, 125.04, 124.83, 122.26, 122.14, 118.88, 114.67, 65.99, 54.29, 53.08, 28.58, 24.19, 23.74; ESI-MS: *m*/*z* =379 [M+H]⁺_{∘}

### Example 4. Synthesis of 1-9 and 1-10 in class I compounds (quinolinones)

### Step 1: 2-(4-(3-Chloropropoxy)phenyl)-3-methoxy-1-methylquinolin-4(1H)-one (Intermediate 10)

Intermediate 9 (660 mg, 2.0 mmol), dimethyl sulfate (0.75 mL, 8.0 mmol) and K₂CO₃ (1.1 g, 8.0 mmol) were dissolved in 10 mL of acetone and heated to reflux for 4 h. When the reaction mixture cooled to ambient temperature, filtered, recovered the solvent. The residue was purified by column chromatography (petroleum ether: EtOAc = 1: 2) to afford 558 mg of white solid. Yield = 78%;¹H NMR (500 MHz, CDCl₃): δ 8.59 (dd, *J=* 8.0, 1.0 Hz, 1H), 7.71 (t, *J* = 7.5 Hz, 1H), 7.53 (d, *J* = 8.5 Hz, 1H), 7.42 (t, *J* = 7.5 Hz, 1H), 7.31 (d, *J* = 8.5 Hz, 2H), 7.07 (d, *J=* 8.5 Hz, 2H), 4.22 (t, *J=* 6.0 Hz, 2H), 3.81 (t, *J=* 6.0 Hz, 2H), 3.65 (s, 3H), 3.54 (s, 3H), 2.36-2.24 (m, 2H); ESI-MS: *m*/*z* =358 [M+H]⁺_{∘}

### Step2: 3-methoxy-1-methyl-2-(4-(3-(pyrrolidin-1-yl)propoxy)phenyl)quinolin-4(1H)-one (1-9)

The preparation method was the same as compound 1-1, and intermediate 10 is used instead of intermediate 4 to obtain off-white solid. Yield = 65%; ¹H NMR (500 MHz, CDCl₃): δ 8.59 (dd, *J* = 8.0, 1.5 Hz, 1H), 7.70 (t, *J* = 80 Hz, 1H), 7.53 (d, *J* = 8.5 Hz, 1H), 7.40 (t, *J* = 7.5 Hz, 1H), 7.28 (d, *J* = 8.0 Hz, 2H), 7.05 (d, *J* = 8.5 Hz, 2H), 4.12 (t, *J* = 6.0 Hz, 2H), 3.65 (s, 3H), 3.53 (s, 3H), 2.70 (t, *J=* 7.5 Hz, 2H), 2.61-2.55 (m, 4H), 2.13-2.05 (m, 2H), 1.85-1.81 (m, 4H); ¹³C NMR (125 MHz, CDCl₃): δ 172.96, 159.69, 147.31, 141.26, 140.20, 131.85, 130.38, 127.14, 126.79, 124.35, 122.99, 115.78, 114.67, 66.46, 59.89, 54.30, 53.15, 37.12, 28.74, 23.47; ESI-MS: *m*/*z* =393 [M+H]⁺_{∘}

### 3-methoxy-1-methyl-2-(4-(3-(piperidin-1-yl)propoxy)phenyl)quinolin-4(1H)-one (I-10)

The preparation method was the same as compound 1-1, using intermediate 10 instead of intermediate 4 and piperidine instead of pyrrolidine to obtain off-white solid. Yield = 67%; ¹H NMR (500 MHz, CDCl₃): δ 8.58 (d, *J=* 8.0 Hz, 1H), 7.70 (t, *J=* 7.5 Hz, 1H), 7.53 (d, *J=* 8.5 Hz, 1H), 7.41 (t, *J* = 7.5 Hz, 1H), 7.29 (d, *J* = 8.5 Hz, 2H), 7.05 (d, *J* = 8.5 Hz, 2H), 4.10 (t, *J* = 6.0 Hz, 2H), 3.65 (s, 3H), 3.53 (s, 3H), 2.57-2.51 (m, 2H), 2.47-2.43 (m, 4H), 2.08-2.03 (m, 2H), 1.66-1.58 (m, 4H), 1.48-1.44 (s, 2H); ¹³C NMR (125 MHz, CDCl₃): δ 172.95, 159.72, 147.28, 141.27, 140.21, 131.82, 130.38, 127.15, 126.79, 124.36, 122.97, 115.77, 114.68, 66.60, 59.88, 55.96, 54.66, 37.10, 26.77, 25.92, 24.39; ESI-MS: *m*/*z* =407 [M+H]⁺_{∘}

### Example 5. Synthesis of II-1 and II-2 in class II compounds (flavonoids)

### Step 1: 4-(3-Chloropropoxy)benzaldehyde (Intermediate 11)

The preparation method was the same as that of intermediate 6, except that p-hydroxybenzaldehyde is used instead of p-hydroxyacetophenone to obtain pale yellow solid. Yield = 98%; ¹H NMR (500 MHz, CDCl₃): δ 9.89 (s, 1H), 7.85 (d, *J=* 9.0 Hz, 2H), 7.02 (d, *J* = 9.0 Hz, 2H), 4.22 (t, *J=* 6.0 Hz, 2H), 3.77 (t, *J=* 6.0 Hz, 2H), 2.30-2.25 (m, 2H); ESI-MS: *m*/*z* =199 [M+H]⁺_{∘}

### Step 2: 1-(2-Hydroxyphenyl)-3-(4-(3-chloropropoxy)phenyl)prop-2-en-1-one (Intermediate 12)

*p*-Hydroxyacetophenone (1.36 g, 10 mmol), intermediate 11 (1.98 g, 10 mmol) were dissolved in 15 mL of C₂H₅OH, 1.68 g of KOH (30 mmol) was added, and the reaction was refluxed for 2 h. When the reaction mixture cooled to ambient temperature, concentrated in vacuo, added 200 mL of ice water to the residue, acidified to pH = 4~5 with 2 N hydrochloric acid. After large amount of solid was precipitated, suction filtered, and dried to obtain 2.82 g yellow solid. Yield = 89%; ¹H NMR (500 MHz, CDCl₃): δ 12.92 (s, 1H), 7.94-7.89 (m, 2H), 7.63 (d, *J* = 8.5 Hz, 2H), 7.57 and 7.54 (s, 1H), 7.49 (t, *J* = 7.5 Hz, 1H), 7.03 (d, *J* = 8.5 Hz, 1H), 6.97-6.93 (m, 3H), 4.20 (t, *J* = 6.0 Hz, 2H), 3.78 (t, *J* = 6.5 Hz, 2H), 2.30-2.25 (m, 2H); ESI-MS: *m*/*z* =317 [M+H]⁺_{∘}

### Step 3: 2-(4-(3-Chloropropoxy)phenyl)-3-hydroxy-4H-chromen-4-one (Intermediate 13)

Intermediate 12 (1.05 g, 3 mmol) was dissolved in 10 mL of C₂H₅OH, 10 mL of 0.5 N KOH solution was added, and 0.6 mL of H₂O₂ aqueous solution (30%) was added in batches, and stirring at ambient temperature for 1 h. The reaction mixture was poured into ice water, and a large amount of solid was precipitated, which was filtered off with suction, and the filter cake was washed with water and dried to obtain 930 mg of yellow solid. Yield = 94%; ¹H NMR (500 MHz, CDCl₃): δ 8.61 (d, *J* = 7.5 Hz, 1H), 8.04 (d, *J=* 9.0 Hz, 2H), 7.61-7.58 (m, 2H), 7.28 (t, *J* = 7.0 Hz, 1H), 7.01 (d, *J* = 9.0 Hz, 2H), 4.15 (t, *J* = 6.0 Hz, 2H), 3.83 (t, *J* = 6.5 Hz, 2H), 2.23-2.17 (m, 2H); ESI-MS: *m*/*z* =331 [M+H]⁺_{∘}

### Step 4: 3-hydroxy-2-(4-(3-(pyrrolidin-1-yl)propoxy)phenyl)-4H-chromen-4-one (II-1)

The preparation method was the same as that of compound 1-1, and intermediate 13 is used instead of intermediate 4 to obtain yellow solid. Yield = 68%; ¹H NMR (500 MHz, CDCl₃): δ 8.25-8.21 (m, 3H), 7.71 (t, *J* = 7.5 Hz, 1H), 7.59 (d, *J* = 8.5 Hz, 1H), 7.42 (t, *J* = 7.5 Hz, 1H), 7.06 (d, *J* = 9.0 Hz, 2H), 4.14 (t, *J* = 6.0 Hz, 2H), 2.74-2.67 (m, 2H), 2.64-2.58 (m, 4H), 2.11-2.06 (m, 2H), 1.86-1.80 (m, 4H); ¹³C NMR (125 MHz, DMSO-*d*₆): δ 173.10, 160.32, 154.88, 146.04, 138.65, 133.96, 129.87, 125.20, 124.95, 123.92, 121.81, 118.80, 114.94, 66.52, 54.10, 52.64, 28.56, 23.56; ESI-MS: *m*/*z* =366 [M+H]⁺_{∘}

### 3-hydroxy-2-(4-(3-(piperidin-1-yl)propoxy)phenyl)-4H-chromen-4-one (II-2)

The preparation method was the same as compound 1-1, using intermediate 13 instead of intermediate 4 and piperidine instead of pyrrolidine to obtain yellow solid. Yield = 61%; ¹H NMR (500 MHz, CDCl₃): δ 8.25-8.21 (m, 3H), 7.71 (t, *J* = 7.5 Hz, 1H), 7.59 (d, *J* = 8.5 Hz, 1H), 7.42 (t, *J* = 7.5 Hz, 1H), 7.06 (d, *J* = 9.0 Hz, 2H), 4.12 (t, *J* = 6.5 Hz, 2H), 2.56 (t, *J* = 7.0 Hz, 2H), 2.46-2.42 (m, 4H), 1.95-1.89 (m, 2H), 1.70-1.67 (m, 4H), 1.52-1.48 (m, 2H); ¹³C NMR (125 MHz, DMSO-*d*₆): δ173.47, 160.24, 154.85, 145.98, 138.02, 133.85, 129.77, 125.19, 124.87, 124.09, 121.78, 118.78, 114.93, 66.61, 55.56, 54.59, 26.69, 26.07, 24.61; ESI-MS: *m*/*z* =380 [M+H]⁺_{∘}

### Example 6. Synthesis of II-3 and II-4 in class II compounds (flavonoids)

### Step 1: 2-(4-(3-Chloropropoxy)phenyl)-3-methoxy-4H-chromen-4-one (Intermediate 14)

Intermediate 13 (660 mg, 2.0 mmol), iodomethane (8.0 mmol) and K₂CO₃ (1.1 g, 8.0 mmol) were dissolved in 10 mL of acetone and heated to reflux for 4 h. After the reaction was completed, cooling and suction filtration, the filtrate was recovered under reduced pressure and the solvent was purified by column chromatography (petroleum ether: EtOAc = 1: 2) to obtain 558 mg of yellow solid. Yield = 78%; ¹H NMR (500 MHz, CDCl₃): δ 8.59 (dd, *J* = 8.0, 1.0 Hz, 1H), 7.71 (t, *J=* 7.5 Hz, 1H), 7.53 (d, *J* = 8.5 Hz, 1H), 7.42 (t, *J* = 7.5 Hz, 1H), 7.31 (d, *J* = 8.5 Hz, 2H), 7.07 (d, *J* = 8.5 Hz, 2H), 4.22 (t, *J* = 6.0 Hz, 2H), 3.81 (t, *J* = 6.0 Hz, 2H), 3.65 (s, 3H), 3.54 (s, 3H), 2.36-2.24 (m, 2H); ESI-MS: *m*/*z* =358 [M+H]⁺_{∘}

### Step 2: 3-methoxy-2-(4-(3-(pyrrolidin-1-yl)propoxy)phenyl)-4H-chromen-4-one (II-3)

The preparation method was the same as compound 1-1, and intermediate 14 is used instead of intermediate 4 to obtain red solid. Yield = 54%; ¹H NMR (500 MHz, CDCl₃): δ 8.26 (d, *J=* 8.0 Hz, 1H), 8.12 (d, *J=* 8.5 Hz, 2H), 7.68-7.64 (m, 1H), 7.53-7.50 (m, 1H), 7.40-7.37 (m, 1H), 7.00 (d, *J* = 8.5 Hz, 2H), 4.12 (t, *J* = 6.0 Hz, 2H), 3.90 (s, 3H), 2.74-2.67 (m, 2H), 2.64-2.58 (m, 4H), 2.11-2.06 (m, 2H), 1.86-1.80 (m, 4H); ¹³C NMR (125 MHz, CDCl₃): δ 175.01, 161.01, 155.72, 155.15, 140.80, 133.28, 130.24, 125.77, 124.58, 124.20, 123.07, 117.90, 114.50, 66.52, 59.92, 54.29, 53.07, 28.68, 23.46; ESI-MS: *m*/*z* =380 [M+H]⁺_{∘}

### 3-methoxy-2-(4-(3-(piperidin-1-yl)propoxy)phenyl)-4H-chromen-4-one (II-4)

The preparation method was the same as compound 1-1, using intermediate 14 instead of intermediate 4 and piperidine instead of pyrrolidine to obtain yellow solid. Yield = 57%; ¹H NMR (500 MHz, CDCl₃): 8.26 (d, *J* = 8.0 Hz, 1H), 8.12 (d, *J* = 8.5 Hz, 2H), 7.68-7.64 (m, 1H), 7.53-7.50 (m, 1H), 7.40-7.37 (m, 1H), 7.00 (d, *J=* 8.5 Hz, 2H), 4.12 (t, *J=* 6.5 Hz, 2H), 3.89 (s, 3H), 2.56 (t, *J=* 7.0 Hz, 2H), 2.46-2.42 (m, 4H), 1.95-1.89 (m, 2H), 1.70-1.67 (m, 4H), 1.52-1.48 (m, 2H); ¹³C NMR (125 MHz, CDCl₃): δ 175.00, 161.03, 155.71, 155.16, 140.82, 133.27, 130.24, 125.80, 124.57, 124.22, 123.09, 117.90, 114.51, 66.65, 59.93, 55.89, 54.67, 26.68, 25.90, 24.38; ESI-MS: *m*/*z* =394 [M+H]⁺_{∘}

### Example 7. Synthesis of II-5 and II-6 in class II compounds (flavonoids)

### Step1: 1-(2,6-Dihydroxyphenyl)-3-(4-(3-chloropropoxy)phenyl)prop-2-en-1-one (Intermediate 15)

The preparation method was the same as that of intermediate 12, and 2,6-dihydroxyacetophenone is used instead of o-hydroxyacetophenone to obtain yellow solid. Yield = 85%; ESI-MS: *m*/*z* =333 [M+H]⁺_{∘}

### Step 2: 2-(4-(3-Chloropropoxy)phenyl)-5-hydroxy-4H-chromen-4-one (Intermediate 16)

Intermediate 15 (1.00 g, 3.2 mmol) was dissolved in 15 mL DMSO, I₂ (128 mg, 0.5 mmol) and 0.5 mL concentrated sulfuric acid were added, and the mixture was stirred at 85 °C for 24 h. The reaction mixture was poured into 200 mL of H₂O, extracted three times with EtOAc, the organic layers were combined, washed with water, saturated NaCl, dried over anhydrous Na₂SO₄. The residue was purified by column chromatography (petroleum ether: EtOAc = 4: 1) to obtain white Solid 565 mg. Yield = 45%; ¹H NMR (500 MHz, CDCl₃): δ 7.88 (d, *J* = 90 Hz, 2H), 7.55 (t, *J* = 8.5 Hz, 1H), 7.05 (d, *J* = 90 Hz, 2H), 7.00 (d, *J* = 80 Hz, 1H), 6.82 (d, *J* = 8.0 Hz, 1H), 6.66 (s, 1H), 4.23 (t, *J* = 6.0 Hz, 2H), 3.79 (t, *J* = 6.0 Hz, 2H), 2.32-2.27 (m, 2H); ESI-MS: *m*/*z* =331 [M+H]⁺_{∘}

### Step 3: 5-hydroxy-2-(4-(3-(pyrrolidin-1-yl)propoxy)phenyl)-4H-chromen-4-one (II-5)

The preparation method was the same as that of compound 1-1, and intermediate 16 is used instead of intermediate 4 to obtain yellow solid. Yield = 58%; ¹H NMR (500 MHz, CDCl₃): δ 7.88 (d, *J=* 9.0 Hz, 2H), 7.55 (t, *J=* 8.5 Hz, 1H), 7.05 (d, *J=* 9.0 Hz, 2H), 7.00 (d, *J* = 8.0 Hz, 1H), 6.82 (d, *J=* 8.0 Hz, 1H), 6.66 (s, 1H), 4.12 (t, *J=* 6.0 Hz, 2H), 2.78 (t, *J=* 6.0 Hz, 2H), 2.65-2.61 (m, 4H), 2.11-2.06 (m, 2H), 1.89-1.86 (m, 4H); ESI-MS: *m*/*z* =366 [M+H]⁺_{∘}

### 5-hydroxy-2-(4-(3-(piperidin-1-yl)propoxy)phenyl)-4H-chromen-4-one (II-6)

The preparation method was the same as compound 1-1, using intermediate 16 instead of intermediate 4 and piperidine instead of pyrrolidine to obtain yellow solid. Yield = 64%; ¹H NMR (500 MHz, CDCl₃): δ 7.87 (d, *J=* 9.0 Hz, 2H), 7.56 (t, *J=* 8.5 Hz, 1H), 7.04 (d, *J=* 9.0 Hz, 2H), 6.99 (d, *J* = 8.0 Hz, 1H), 6.81 (d, *J=* 8.0 Hz, 1H), 6.65 (s, 1H), 4.13 (t, *J=* 6.0 Hz, 2H), 2.59-2.53 (m, 2H), 2.50-2.44 (m, 4H), 2.10-2.04 (m, 2H), 1.70-1.62 (m, 4H), 1.48-1.45 (m, 2H); ESI-MS: *m*/*z* =380 [M+H]⁺_{∘}

### Example 8. Synthesis of II-7 and II-8 in class II compounds (flavonoids)

### Step 1: 2-(4-(3-Chloropropoxy)phenyl)-5-methoxy-4H-chromen-4-one (Intermediate 17)

The preparation method was the same as that of intermediate 14, and intermediate 16 is used instead of intermediate 13 to obtain off-white solid. Yield = 75%; ¹H NMR (500 MHz, CDCl₃): δ7.88 (d, *J=* 9.0 Hz, 2H), 7.55 (t, *J=* 8.5 Hz, 1H), 7.05 (d, *J=* 9.0 Hz, 2H), 6.99 (d, *J* = 8.0 Hz, 1H), 6.82 (d, *J* = 8.0 Hz, 1H), 6.68 (s, 1H), 4.21 (t, *J* = 6.0 Hz, 2H), 3.90 (s, 3H), 3.78 (t, *J=* 6.0 Hz, 2H), 2.32-2.27 (m, 2H); ESI-MS: *m*/*z* =345 [M+H]⁺_{∘}

### Step 2: 5-methoxy-2-(4-(3-(pyrrolidin-1-yl)propoxy)phenyl)-4H-chromen-4-one (II-7)

The preparation method was the same as compound 1-1, and intermediate 17 is used instead of intermediate 4 to obtain yellow solid. Yield = 61%; ¹H NMR (500 MHz, CDCl₃): δ7.87 (d, *J=* 9.0 Hz, 2H), 7.55 (t, *J=* 8.5 Hz, 1H), 7.05 (d, *J=* 9.0 Hz, 2H), 6.99 (d, *J=* 8.0 Hz, 1H), 6.82 (d, *J=* 8.0 Hz, 1H), 6.68 (s, 1H), 4.13 (t, *J=* 6.0 Hz, 2H), 3.91 (s, 3H), 2.76 (t, *J* = 6.0 Hz, 2H), 2.65-2.60 (m, 4H), 2.10-2.05 (m, 2H), 1.89-1.85 (m, 4H); ESI-MS: *m*/*z* =380 [M+H]⁺_{∘}

### 5-methoxy-2-(4-(3-(piperidin-1-yl)propoxy)phenyl)-4H-chromen-4-one (II-8)

The preparation method was the same as compound 1-1, using intermediate 17 instead of intermediate 4 and piperidine instead of pyrrolidine to obtain yellow solid. Yield = 67%; ¹H NMR (500 MHz, CDCl₃): δ 7.88 (d, *J=* 9.0 Hz, 2H), 7.56 (t, *J=* 8.5 Hz, 1H), 7.05 (d, *J=* 9.0 Hz, 2H), 7.00 (d, *J* = 8.0 Hz, 1H), 6.83 (d, *J* = 8.0 Hz, 1H), 6.66 (s, 1H), 4.12 (t, *J* = 6.0 Hz, 2H), 3.89 (s, 3H), 2.54-2.49 (m, 2H), 2.48-2.41 (m, 4H), 2.06-2.00 (m, 2H), 1.65-1.59 (m, 4H), 1.47-1.43 (m, 2H); ESI-MS: *m*/*z* =394 [M+H]⁺_{∘}

### Example 9. The relation between the dosage of Compound 1-6 and the heat pain threshold of PSL model

Male SD rats, weighting 200-250g, were used to establish partial sciatic nerve ligation (PSL) model. The rats were subjected to ligation of the right sciatic nerve at high-tight level, so that 1/3-1/2 thickness of the sciatic nerve was trapped in the ligature, and then the wound was closed. The rats in sham group were subjected to the same operative procedure as the PSL model but without nerve ligation. On the sixth day after operation, heat pain threshold was measured in PSL model rats with Plantar Test Apparatus (BME-410) for 2 days to choose rats with stable heat pain threshold, which were randomly divided into 5 groups: 1) model; 2) 30 mg/kg Pregabalin; 3) 3 mg/kg Compound 1-6; 4) 1 mg/kg Compound 1-6; and 5) 0.3 mg/kg Compound 1-6. Group 2 -5 were given corresponding medicines orally with the dose of 1ml/100g, while the Group model and normal were given saline of the same volume with the same method. Heat pain threshold was measured before administration and 1, 2, 4, 6h after intragastric administration, and the results were shown in Figure 1.

As shown in Figure 1, in PSL model rats the heat pain threshold increased in a dose-dependent manner after administrating single dose of 0.3 mg/kg, 1 mg/kg or 3 mg/kg Compound 1-6. The heat pain threshold of 0.3 mg/kg Compound 1-6 group was significantly higher (P<0.01) than model group at the 2nd hour after administration. The heat pain threshold of 1.0 mg/kg Compound 1-6 group was raised to be compared to 30 mg/kg Pregabalin group. The heat pain threshold of 3.0 mg/kg Compound 1-6 group was further improved to the level equal to sham group during the 1st-2nd hour after administration, which was better than Pregabalin group.

### Example 10. The effect of different drugs on heat pain threshold of PSL model

Male SD rats, weighting 200-250g, were used to establish partial sciatic nerve ligation (PSL) model. The rats were subjected to ligation of the right sciatic nerve at high-tight level, so that 1/3-1/2 thickness of the sciatic nerve was trapped in the ligature, and then the wound was closed. The rats in sham group were subjected to the same operative procedure as the PSL model but without nerve ligation. On the sixth day after operation, heat pain threshold was measured in PSL model rats with Plantar Test Apparatus (BME-410) for 2 days to choose rats with stable heat pain threshold, which were randomly divided into 7 groups: 1) model; 2) 50 mg/kg Gabapentin; 3) 100 mg/kg Carbamazepine; 4) 10 mg/kg Amitriptyline; 5) 1 mg/kg Compound 1-4; 6) 1 mg/kg Compound 1-6; and 7) 1 mg/kg Compound II-4. Group 2 -7 were given corresponding medicines orally with the dose of 1ml/100g, while the Group model and normal were given saline of the same volume with the same method. Heat pain threshold was measured before and 1, 2, 4, 6h after intragastric administration, and the results were shown in Figure 2.

As shown in Figure 2, in PSL model rats the heat pain threshold of 1 mg/kg Compound 1-4 group, 1 mg/kg Compound 1-6 group or 1 mg/kg Compound II-4 group was significantly higher than model group since 1 hour after administration, and was compared to 50 mg/kg Gabapentin group, 100 mg/kg Carbamazepine group or 10 mg/kg Amitriptyline group. These results suggested the efficacy of compounds mentioned above.

### Example 11. The relation between the dosage of Compound 1-4 and the mechanical withdrawal threshold of CCI model

Male SD rats, weighting 200-250g, were used to establish chronic constrictive injury (CCI) model. The common sciatic nerve was exposed at the level of the middle of the thigh, and 4 ligatures (4.0 chromic gut, 0.15mm) were tied loosely with about 1-2mm spacing to the position proximal to the sciatic's trifurcation. The rats in sham group were subjected to the same operative procedure as the CCI model but without nerve ligation. On the seventh day after operation, mechanical withdrawal threshold was measured in CCI model rats with Electric Von Frey (IITC-2391) for 2 days to choose rats with stable mechanical withdrawal threshold, which were divided into 5 groups: 1) model; 2) 50 mg/kg Gabapentin; 3) 9 mg/kg Compound 1-4; 4) 3 mg/kg Compound 1-4; and 5) 1 mg/kg Compound 1-4. Group 2 -5 were given corresponding medicines orally with the dose of 1ml/100g, while the Group model and normal were given saline of the same volume with the same method. Mechanical withdrawal threshold was measured before administration and 1, 2, 4, 6h after intragastric administration, and the results were shown in Figure 3.

As shown in Figure 3, in CCI model rats the mechanical withdrawal threshold exhibited a dose-dependent increase after administration of 1 mg/kg, 3 mg/kg or 9 mg/kg Compound 1-4. The mechanical withdrawal threshold of 3 mg/kg Compound 1-4 group was significantly higher (P<0.01) than model group during 1st-4th hour after administration. While the mechanical withdrawal threshold of 50 mg/kg Gabapentin group only remained significantly higher than model group during 1st-2nd hour after administration. These results indicated that the effective time of Compound 1-4 was longer than Gabapentin.

### Example 12. The effect on diabetic neuropathic pain model

Male SD rats (200-250g) with normal blood sugar were fasted but had free access to water for 12 hours. The diabetes model was induced by intraperitoneal injection of 60 mg/kg streptozotocin (STZ), and was examined by 2-hour postprandial sugar level on day 7 after injection, which should be higher than 16 mmol/L. The rats in normal group were given an equal volume of citric acid-sodium citrate buffer (pH 4.2). On day 14 after injection, the heat pain threshold of rats in diabetes model or normal group were measured with Plantar Test Apparatus (BME-410) to examine the success of diabetic neuropathic pain model. The rats in diabetic neuropathic pain model were divided into 3 groups: 1) model; 2) 50 mg/kg Gabapentin; and 3) 1mg/kg Compound 1-6. Group 2 and 3 were given corresponding medicines orally with the dose of 1ml/100g, while the Group model and normal were given saline of the same volume with the same method. Heat pain threshold was measured before administration and 1, 2, 4, 6h after administration, and the results were shown in Figure 4.

As shown in Figure 4, both Compound 1-6 and Gabapentin could improve the heat pain threshold of diabetic neuropathic pain model, but by contrast, 1mg/kg Compound 1-6 showed significantly higher heat pain threshold (P<0.01) and longer effective time.

### Example 13. The effect on chemotherapy-induced neuropathic pain model

Male SD rats (200-250g) were injected 4mg/kg of Oxaliplatin by intraperitoneal on day 1, 2, 8, 9, 15, 16, 22 and 23 to establish chemotherapy-induced neuropathic pain model. On day 24, the mechanical withdrawal threshold of rats in group normal and model were measured with Electric Von Frey (IITC-2391) to choose rats with stable mechanical withdrawal threshold, which were randomly divided into 5 groups: 1) model; 2) 30 mg/kg Pregabalin; 3) 9 mg/kg Compound II-4; 4) 3 mg/kg Compound II-4; and 5) 1 mg/kg Compound II-4. Group 2 -5 were given corresponding medicines orally with the dose of 1ml/100g, while the Group model and normal were given saline of the same volume with the same method. Mechanical withdrawal threshold was measured before administration and 1, 2, 4, 6h after single administration, and the results were shown in Figure 5.

As shown in Figure 5, in chemotherapy-induced neuropathic pain model, the mechanical withdrawal threshold of OXA neuropathic pain model rats was dosage-dependent increase after administrating single dose of 1 mg/kg, 3 mg/kg or 9 mg/kg Compound II-4. The mechanical withdrawal threshold of 1 mg/kg Compound II-4 was significantly higher (P<0.01) than model group during 1st-2nd hour after administration. The mechanical withdrawal thresholds of 3 mg/kg Compound II-4 or 9 mg/kg Compound II-4 was significantly higher (P<0.05) than 30 mg/kg Pregabalin during 2nd-4th hour after administration. Therefore, although Pregabalin was of rapid action, Compound II-4 could improve mechanical withdrawal threshold of OXA neuropathic pain model more effectively and longer.

### Example 14. The effect of 5 days repeated administration on the mechanical withdrawal threshold of CCI model

Male SD rats, weighting 200-250g, were used to establish CCI model. The common sciatic nerve was exposed at the level of the middle of the thigh, and 4 ligatures (4.0 chromic gut, 0.15mm) were tied loosely with about 1-2mm spacing to the position proximal to the sciatic's trifurcation. The rats in sham group were subjected to the same operative procedure as the CCI model but without nerve ligation. On the seventh day after operation, mechanical withdrawal threshold was measured in CCI model rats with Electric Von Frey (IITC-2391) for 2 days to choose rats with stable mechanical withdrawal threshold, which were divided into 5 groups: 1) model; 2) 50 mg/kg Gabapentin; 3) 3 mg/kg Compound 1-10; 4) 1 mg/kg Compound I-10; and 5) 0.3 mg/kg Compound I-10. Group 2 -5 were given corresponding medicines orally with the dose of 1ml/100g for 5 days, while the Group model and normal were given saline of the same volume with the same method. Mechanical withdrawal threshold was measured before and 2h after intragastric administration, and the results were shown in Figure 6.

As shown in Figure 6, in CCI model rats the mechanical withdrawal threshold increased in a dose-dependent manner after repeated administration of 0.3 mg/kg, 1 mg/kg or 3 mg/kg Compound I-10. The mechanical withdrawal threshold of 1 mg/kg Compound I-10 group was significantly higher (P<0.01) than model group during 1st -5th day after administration, while 50 mg/kg Gabapentin only kept the significant difference during 2nd -5th day after administration, so that Compound I-10 showed better dose-dependency and stability of efficacy compared to Gabapentin.

### Example 15. Bioavailability

The in vivo pharmacokinetic studies of Compound 1-6 were conducted in SD rats (n=3). The results showed that after I.V administration of 1.00mg/kg Compound 1-6, the half-life was 6.00 ± 1.43 h; after P.O administration of 10.0 mg/kg Compound 1-6, peak plasma concentrations occur at 2.00 ± 1.73 h, and the half-life was 3.79 ± 0.12 h; and the absolute bioavailability of Compound 1-6 in SD rats was 51.32%.

### Example 16. Security

ICR mice (n=4 in each group) were given different dosage of Compound 1-6 by intragastric administration. There was no abnormal signs of the mice given a dose of 64 mg/kg; there was one of the mice given a dose of 256 mg/kg suffered convulsions but recovered on the second day; and there was one of the mice given a dose of 1024 mg/kg suffered convulsions and finally died on the second day. The half lethal dose (LD₅₀) of Compound 1-6 has not been obtained, but it should be higher than 1024mg/kg, indicating its good security.

## Claims

1. Phenylquinolinone deriviative or flavonoid derivative for use in the treatment of neuropathic pain diseases, wherein said phenylquinolinone deriviative or flavonoid derivative is used in preparation of a medicament for the treatment of neuropathic pain diseases, the phenylquinolinone derivative or flavonoid derivative has a general structural formula as following: 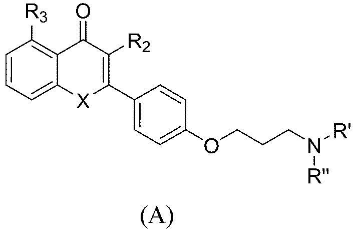 Wherein:
X is 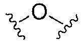 OR 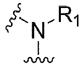 , R₁ is hydrogen or selected from a group consisting of C₁₋₃ alkyl and C₁₋₃ alkoxy;
R₂ is hydrogen or selected from a group consisting of hydroxy and C₁₋₃ alkoxy;
R₃ is hydrogen or selected from a group consisting of hydroxy, C₁₋₃ alkyl and C₁₋₃ alkoxy;
NR'R" is selected from a group of cyclic amines with 3- to 6-membered carbon atoms and open chain alkylamines with 3- to 6-membered carbon atoms, the group including the following segments: 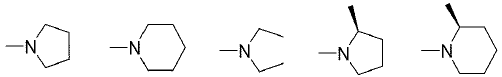
wherein said phenylquinolinone derivative or flavonoid derivative comprises pharmaceutically acceptable salts.

2. The use according to claim 1, wherein said phenylquinolinone derivative or flavonoid derivative is selected from compounds in the following table:
Entry Structure Entry Structure 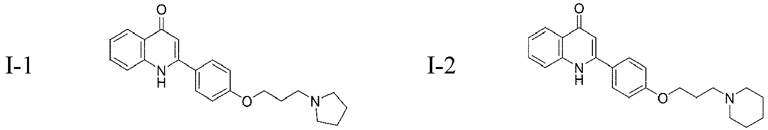 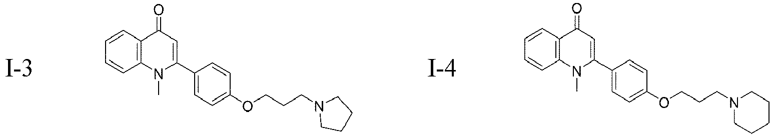 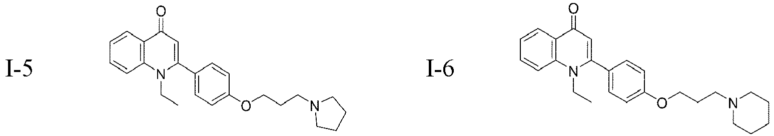 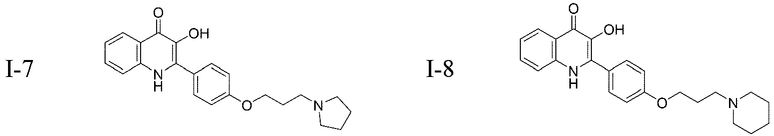 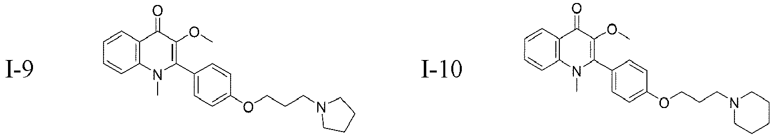 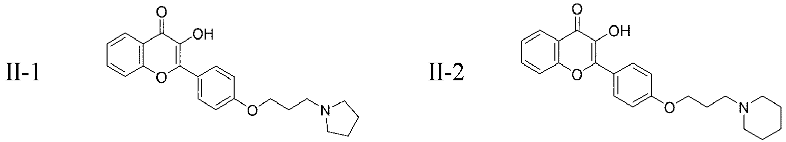 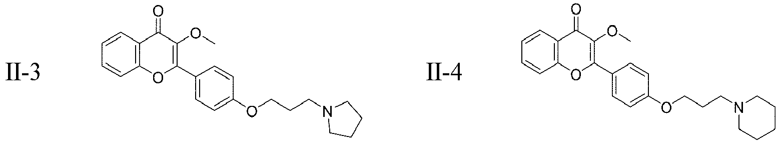 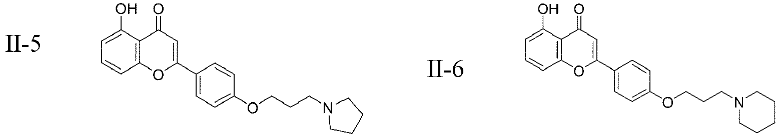 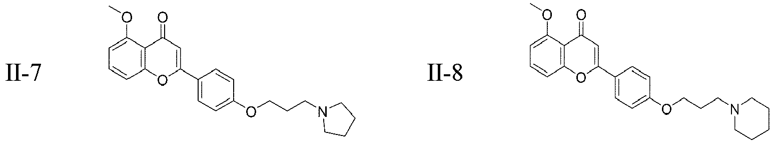 or a pharmaceutically acceptable salt of said compounds.

3. The use according to claim 1 or claim 2, wherein said salts are hydrochloride, sulfate, phosphate, hydrobromide, hydroiodide, carbonate, citrate, malate, tartrate, oxalate, lactate, malonate, succinate, glutarate, ketoglutarate, ascorbate, fumarate, maleate, mesylate, *p*-toluenesulfonate or benzenesulfonate.

4. The use according to any one of claims 1 to 3, wherein said neuropathic pain disease is peripheral neuropathic pain or central neuropathic pain.

5. The use according to claim 4, wherein said peripheral neuropathic pain is one selected from trigeminal neuralgia, glossopharyngeal neuralgia, acute or chronic inflammatory demyelinating polyneuropathy, alcoholic polyneuralgia, chemotherapy-induced polyneuralgia, complex regional pain syndrome symptoms, entrapment neuralgia, HIV sensory neuralgia, iatrogenic neuralgia, tumor compression or infiltration neuralgia, dystrophic neuralgia, diabetic neuralgia, phantom limb pain, postherapetic neuralgia, Post-radiotherapy plexopathy, radiculopathy, poison exposure-related neuralgia, or post-traumatic neuralgia.

6. The use according to claim 4, wherein said central neuropathic pain is one selected from post-stroke pain, multiple sclerosis related pain, Parkinson disease related pain, post-traumatic spinal cord injury pain, syringomyelia, post-ischemic myelopathy, compressive myelopathy, HIV myelopathy, or post-radiation myelopathy.

7. The use according to claim 1, wherein the administration of the drug is oral administration, rectal administration, nasal administration, topical administration or parenteral administration.

8. The use according to claim 7, wherein said topical administration is selected from buccal, sublingual or transdermal administration; the parenteral administration is selected from subcutaneous injection, intramuscular injection, intravenous injection or intradermal injection.
